# EUROPÄISCHE PATENTSCHRIFT

(11) **EP 0 599 187 B1**
(45) Veröffentlichungstag und Bekanntmachung des Hinweises auf die Patenterteilung: **30.09.1998**
(21) Anmeldenummer: 93118527.6
(22) Anmeldetag: 17.11.1993
(51) Int. Cl.: A61C 13/00

(54) **Verfahren zur Herstellung von Zahnersatz auf Keramikbasis**
Method for making dental prostheses from a ceramic material
Procédé de fabrication de prothèses dentaires à base de céramiques

(30) Priorität: 25.11.1992 DE 4239549; 12.12.1992 DE 4242007
(43) Veröffentlichungstag der Anmeldung: 01.06.1994
(73) Patentinhaber: Vita Zahnfabrik H. Rauter GmbH & Co KG, D-79713 Bad Säckingen (DE)
(72) Erfinder: Thiel, Norbert, Dr., D-79713 Bad Säckingen (DE); Datzmann, Gabriele, D-79713 Bad Säckingen (DE)
(74) Vertreter: Meyers, Hans-Wilhelm, Dr.Dipl.-Chem.

(56) Entgegenhaltungen:
- EP-A- 0 311 214
- EP-A- 0 477 157
- Duret, Funkschau 18/1986, S. 7
- JADA Articles, Vol. 117, Nov. 1988, p. 715-720, F. Duret et al, CAD-CAM in dentistry.

## Beschreibung

Gegenstand der vorliegenden Erfindung ist ein Verfahren zur Herstellung von Zahnersatz auf Metalloxidbasis gemäß dem Oberbegriff von Anspruch 1.

Ein Verfahren, welches die Merkmale des Oberbegriffs von Anspruch 1 aufweist, ist aus dem Bericht von F. Duret, Grenoble, Funkschau 18/1986, Seite 7 bekannt.

Zahnersatz sind im allgemeinen Teile, die auf das natürliche Gebiß eines Patienten gesetzt werden, um dieses mit seiner natürlichen Funktion möglichst weitgehend wieder herzustellen. Diese Teile werden in sehr aufwendigen Verfahren hergestellt. Zunächst muß vom Zahnarzt der zu ersetzende Teil des Gebisses, z. B. einzelne oder mehrere Zähne, präpariert oder extrahiert werden. Nach Anfertigung eines Modells, z. B. durch Erstellen eines Abdrucks mit herkömmlichen Verfahren, wird dann vom Zahnarzt oder Zahntechniker der entsprechende Zahnersatz hergestellt. Dazu gehören Kronen, Brücken, In- und Onlays und andere.

Um einen zufriedenstellenden ästhetischen Eindruck des Zahnersatzes zu schaffen, kann dieser mit Materialien verblendet werden, die gute mechanische Eigenschaften besitzen und gleichzeitig den Farbeindruck natürlicher Zähne vermitteln. Als Material zur Verblendung von Zahnersatz oder Kronen usw. hat sich insbesondere Keramik bewährt.

Als Träger der Verblendung kommen nach dem Stand der Technik insbesondere Metallgerüste in Betracht, die aber neben dem Nachteil der aufwendigen und teuren Herstellung zumindest in Einzelfällen für den Patienten unverträglich sind.

Einige Nachteile von Restaurationen mit metallischen Gerüsten, wurde bereits durch die Verwendung von Kernmaterialien aus Keramik umgangen.

So wird in der EP-A-0 240 643 bzw. der US-A-4 772 436 ein Verfahren beschrieben zur Herstellung von keramischen Restaurationen wie Brücken, Kronen, Inlays, Onlays etc., wobei zunächst ein Meistermodell angefertigt wird. Dieses Modell wird dubliert und der so erhaltene Abdruck mit einem Spezialgips ausgegossen. Auf das entformte Gipsgerüst wird ein feinkörniger oxidkeramischer Schlicker aufgetragen. Dabei entzieht der Gipsstumpf sofort den Flüssigkeitsanteil und es entsteht eine fast trockene Masseschicht, die sich gut zu dem gewünschten Gerüst ausarbeiten läßt. Das vorliegende Gerüst wird mit dem Gipsstumpf in dem Temperaturbereich gesintert, in dem noch keine Schwindung eintritt. Zur Erzielung der hohen Biegefestigkeit wird das poröse Gerüst in einem Infiltrationsbrand mit Glas getränkt.

Dabei ist die Verarbeitung in hohem Maße von der Geschicklichkeit des Zahntechnikers abhängig. Außerdem ist eine Nachbearbeitung des Gerüstes durch seine hohe Festigkeit und Zähigkeit kaum möglich.

Duret beschreibt die Verwendung von CAD/CAM-Methoden zur Konstruktion und Fertigung von Zahnprothesen. Dabei wird ein Zahnstumpf in seiner Umgebung aus mehreren Richtungen mit einer Videokamera aufgenommen, die Aufnahmen digitalisiert und einen CAD-fähigen Computer in eine dreidimensionale Abbildung umgerechnet. Der Zahnersatz läßt sich auf dem Bildschirm gemäß gespeicherten Modellen auf dem Stumpf konstruieren. Die Prothese wird dann durch eine computergesteuerte Mikrofräse aus einem vorgefertigten Block des gewählten Materials, Metall oder Keramik, herausgearbeitet. Die dabei verwendeten Materialien sind im Falle von Keramikmaterialien solche, die nicht die optimale Festigkeit erreichen.

Die DD 261,741 A1 beschreibt ein Verfahren, wobei ebenfalls Inlays, die aus Glaskeramik bestehen, ihre Form durch maschinelle Bearbeitung erhalten. Diese maschinelle Bearbeitung kann mit Hilfe von elektrooptischen Abtastungen des Zahnes oder dem Kopierfräsen erfolgen. Die dabei zu verwendende Glaskeramik weist nicht die hohen Festigkeitswerte auf, die mit modernen Keramikmaterialien erreichbar sind, aber aufgrund der Härte maschinell nicht mit vertretbarem Aufwand bearbeitbar sind.

Die EP 0 311 214 beschreibt ein Verfahren zur Herstellung einer Zahnkrone für einen dafür vorbereiteten Zahn mit Hilfe eines CAD/CAM-Systems, wobei unter der Kontrolle des CAD/CAM-Systems ein Modell des vorbereiteten Zahns aus einem feuerfesten Material gemacht wird. Auf das Modell wird sukzessiv ein oder mehrere Schichten eines geeigneten Materials aufgetragen, wobei jede Schicht nach ihrer Auftragung gesintert und anschließend unter der Kontrolle des CAD/CAM-Systems geschliffen wird.

Die EP 0 477 157 beschreibt ein Verfahren zur Herstellung von Kunstzahnveneers bestehend aus einem Keramik- oder anderen Kern, der nach der Pulvertechnologie zur Veneerrestauration gebrannt wird, wobei die innere Fläche des Kerns, welche zur vorbereiteten Zahnoberfläche passen soll, durch Pressen, Vorsintern und Sintern entsteht. Dabei ist in einigen Fällen mit starker Schrumpfung zu rechnen.

Aufgabe der vorliegenden Erfindung ist es somit ein Verfahren anzugeben, mit dem Zahnersatz auf Keramikbasis wirtschaftlicher hergestellt werden kann und Materialien in Form von Rohlingen, die unter optimalen Bedingungen industriell gefertigt werden, zum Einsatz kommen. Dabei sollen die Vorteile der modernen oxidkeramischen Werkstoffe mit sehr hoher mechanischer Festigkeit genutzt werden können.

Diese Aufgabe wird gelöst durch ein Verfahren mit den Merkmalen des Anspruchs 1.

Als Formkörper (Rohlinge), die im erfindungsgemäßen Verfahren zum Einsatz kommen können, sind Rohlinge zu nennen, die aus Keramiken auf der Basis von Aluminiumoxid, Spinell, Titanoxid, Zirkonoxid oder Kombinationen davon sowie geringer Mengen silikatischer Zusätze hergestellt worden sind. Die Rohlinge können in Anlehnung an die Verfahrensweise wie sie in der EP-A-0 240 643 bzw. der US-A-4 772 436 beschrieben ist, hergestellt werden.

Zur Herstellung der Formkörper kann Metalloxidpulver in vorgegebenen Formen gegossen bzw. gepreßt werden, aus denen dann entsprechende Blöcke porös gesintert werden. Wird das pulverförmige Material mit einem Bindemittel versetzt zur besseren Formbarkeit, wird dieses vorzugsweise ausgebrannt, wonach dann die Sinterung erfolgt und
der Zahnersatz direkt herausgearbeitet und anschließend durch Glasinfiltration verfestigt wird.

Die Formkörper (Rohlinge) sind zur besseren Bearbeitbarkeit, vorzugsweise mit einer Halteeinrichtung versehen, beispielsweise ein im Formkörper verankerter, aus demselben herausragender Stift. Der Stift kann zum Beispiel in einer Kontaktfräseeinrichtung eingesetzt werden und fixiert den Rohling bei der Bearbeitung.

Vorzugsweise erfolgt die mechanische Bearbeitung des Rohlings durch eine Schleif- und/oder Frästechnik mit CAD/CAM unterstützten Verfahren bzw. durch Kopierschleifverfahren. Dabei ist es möglich, die präparierten Zähne im Mund fotografisch oder digital oder mittels konventioneller Methoden zu erfassen. Bei konventioneller Vorgehensweise wird zunächst von dem entsprechend präparierten Zahn oder Bereich des Gebisses ein Abdruck genommen, der dann direkt kopiert oder durch Laser digitalisiert und in einem Computer eingelesen werden kann. Diese Daten dienen dann der rechnergestützten Anfertigung des genau an die Bedürfnisse des Patienten angepaßten Zahnersatzes wie Kappen, In- und Onlays, Brücken etc.

Es ist jedoch auch möglich die Situation im Patienten durch fotografische Aufnahme und Einspeisung entsprechend digitalisierter Daten zu erfassen. Ebenso möglich ist die direkte Übernahme der Situation im Munde des Patienten ebenfalls beispielsweise durch Laser- oder Kontaktkopieren.

Die EP 0 455 855 beschreibt zum Beispiel eine Vorrichtung und ein Verfahren zur Erstellung von zahnmedizinischen Prothesen und Paßkörpern.

Diese Vorgehensweise hat den Vorteil, daß ausgehend von standardisiertem Material, wie es der insbesondere industriell vorgefertigte Rohling darstellt, direkt der geformte Körper für die entsprechende Restauration hergestellt werden kann, praktisch maßgeschneidert auf die Situation des Patienten ohne die recht komplexen und störungsanfälligen Dublierschritte zu durchlaufen, wie sie in der herkömmlichen Zahntechnik erforderlich sind.

Wenn die gewünschten, an die Situation des Patienten angepaßten Formteile auf Keramikbasis anhand der Vorlage gefräst und geschliffen worden sind, erfolgt die Behandlung des Formteils mit der Glasschmelze. Dies geschieht ebenfalls wie in der EP-A-240 643 bzw. der US-A-4 772 436 beschrieben. Dabei soll das Glas die Eigenschaft besitzen, das Keramikformteil vollständig zu benetzen. Die entsprechenden Benetzungseigenschaften des Glases können durch Einführung von Zuschlagsstoffen wie Boroxid, Bleioxid oder Vanadiumoxid variiert werden. Bevorzugt ist die Verwendung von solchen Gläsern, deren Ausdehnungskoeffizient unter oder nahe desjenigen des Keramikformteiles liegt, so daß eine zufriedenstellende Stabilität der dentalen Restauration gegenüber thermischem Schock gegeben ist. Die Infiltration erfolgt beispielsweise bei ca. 1.100°C, wobei das geschmolzene Glas in die Kapillaren des Keramikformteiles eindringen kann.

Daran kann sich dann eine Verblendung des infiltrierten Keramikformteiles anschließen. Dies kann beispielsweise mit einer oder mehreren Keramikschichten erfolgen, die verschiedene optische Eigenschaften und Tönungen haben, so daß der Restauration das Aussehen natürlicher Zähne gegeben werden kann. Selbstverständlich sind diese Verfahrenschritte auch möglich für Teilrestaurationen wie Inlays, Onlays, Brücken etc.

Das erfindungsgemäße Verfahren ermöglicht bei Anwendung von CAD/CAM-Verfahren einen rechnerischen Vorhalt für die spätere keramische Verblendung mit z. B. Vitadur® α. Dies ist insbesondere auch bei der Verwendung von Spinell als keramischem Kernmaterial möglich. Auf solche Art hergestellte Formteile kann dann die Verblendung auch durch Aufsprühen von bekannten Materialien wie Vita-Dur, -Dentin, -Opakdentin und -Schmelz mit dem Spray-on-System und anschließendem Brennen durchgeführt werden. Damit lassen sich gute mechanische Werte (infiltriertes Spinell 300 bis 400 MPa) natürliche Transluzenz der gesamten Arbeit, gute Paßgenauigkeit und einfaches, schnelles Verblenden realisieren.

Insgesamt ermöglicht das erfindungsgemäße Verfahren die folgenden Vorteile gegenüber den Verfahren aus dem Stand der Technik, nämlich hervorragende maschinelle Bearbeitungsmöglichkeit des porösen Formkörpers, nach Infiltration hohe mechanische Festigkeit im Dreipunkt-Biegetest. Diese beträgt für infiltriertes Aluminiumoxid etwa 450 bis 500 MPa und für die Kombination Aluminiumoxid/Zirkonoxid 500 bis 700 MPa.

Daraus ergeben sich als mögliche Einsatzgebiete die Einzelkronenpräparation im gesamten Kiefer, Brücken im Frontzahn- und prämolaren Gebiet, wo es ganz besonders auf ästhetische Wirkungen ankommt. Durch die Fabrikvorfertigung der Metalloxidformteile werden mögliche zahntechnisch bedingte individuelle Fehler weitgehend vermieden. Die Kombination mit Verblendkeramik liefert gute ästhetische Wirkung.

## Patentansprüche

1. Verfahren zur Herstellung von Zahnersatz auf Metalloxidbasis bei dem ein vorgefertigter Rohling aus Keramik unter Berücksichtigung der Situation am Patienten maschinell bearbeitet wird, dadurch gekennzeichnet, daß der vorgefertigte Rohling aus porösen oxidkeramischen Werkstoffen, wie Aluminiumoxid, Magnesiumoxid, Zirkonoxid, Titanoxid und deren Kombinationen sowie geringen Mengen silikatischer Zusätze besteht, und daß der aus dem porösen Rohling auf Keramikbasis erhaltene bearbeitete Formkörper durch Behandlung mit einer Glasschmelze verfestigt wird.

2. Verfahren nach Anspruch 1, wobei im Falle von Mischoxiden diese im Gefüge vorliegen, insbesondere als Spinell.

3. Verfahren nach mindestens einem der Ansprüche 1 und/oder 2, wobei die Bearbeitung des Rohlings maschinell mit Unterstützung durch geeignete Verfahren, wie CAD/CAM-Verfahren, erfolgt.

4. Verfahren nach mindestens einem der Ansprüche 1 bis 3, wobei die Situation am Patienten durch vorher gewonnenen Abdruck nachgestellt wird und durch geeignete Verfahren, wie CAD/CAM-Verfahren, der Rohling angepaßt wird.

5. Verfahren nach mindestens einem der Ansprüche 1 bis 4, wobei die Situation am Patienten direkt "online" erstellt wird und über CAD/CAM-Verfahren der Rohling angepaßt wird.

6. Verfahren nach mindestens einem der Ansprüche 1 bis 5, wobei der Zahnersatz Brücken, Kappen, Inlay, Onlay, Veneer oder Teile derselben ist.

7. Verfahren nach mindestens einem der Ansprüche 1 bis 6, wobei der verfestigte Keramikformkörper mit an sich bekannten Verfahren verblendet wird.

## Claims

1. A method for the preparation of metal-oxide based dental prostheses in which a prefabricated blank of a ceramic material is machined taking into account the situation relating to the patient, characterized in that said prefabricated blank consists of porous oxide-ceramic materials, such as alumina, magnesia, zirconia, titania and their combinations as well as small amounts of added silicates, and that the machined part obtained from said ceramic-based porous blank is consolidated by treatment with a glass melt.

2. The method according to claim 1 wherein mixed oxides, when used, form a common crystal structure, especially a spinel structure.

3. The method according to at least one of claims 1 and/or 2, wherein said machining of the blank is effected with the aid of appropriate methods, such as CAD/CAM methods.

4. The method according to at least one of claims 1 to 3, wherein the situation relating to the patient is modelled by a previously prepared impression, and the blank is fitted by appropriate methods, such as CAD/CAM methods.

5. The method according to at least one of claims 1 to 4, wherein the situation relating to the patient is directly established "on line", and the blank is fitted by CAD/CAM methods.

6. The method according to at least one of claims 1 to 5, wherein said dental prostheses are bridges, caps, inlay, onlay, veneer or parts thereof.

7. The method according to at least one of claims 1 to 6, wherein the consolidated ceramic part is faced by per se known methods.

## Revendications

1. Procédé de fabrication d'une prothèse dentaire à base d'oxyde de métal, dans lequel on usine à la machine une ébauche préfabriquée en céramique en prenant en considération l'assise dans la bouche du patient, caractérisé en ce que l'ébauche préfabriquée est formée de matériaux poreux en oxyde céramique, comme l'oxyde d'aluminium, l'oxyde de magnésium, l'oxyde de zirconium, l'oxyde de titane et leurs combinaisons, ainsi que de petites quantités d'additifs silicatés, et en ce qu'on consolide le corps de forme usiné obtenu à partir de l'ébauche poreuse à base de céramique par traitement avec du verre fondu.

2. Procédé selon la revendication 1, dans lequel, dans le cas d'oxydes mixtes, ceux-ci sont présents dans la structure, en particulier sous la forme de spinelle.

3. Procédé selon au moins l'une des revendications 1 et/ou 2, dans lequel on effectue l'usinage à la machine de l'ébauche à l'aide de procédés appropriés, comme un procédé CAD/CAM.

4. Procédé selon au moins l'une des revendications 1 à 3, dans lequel on rajuste l'assise dans la bouche du patient au moyen d'une empreinte préalable et on adapte l'ébauche au moyen de procédés appropriés, comme un procédé CAD/CAM.

5. Procédé selon au moins l'une des revendications 1 à 4, dans lequel on établit l'assise dans la bouche du patient en direct et on adapte l'ébauche au moyen d'un procédé CAD/CAM.

6. Procédé selon au moins l'une des revendications 1 à 5, dans lequel la prothèse dentaire est un bridge, une coiffe, une incrustation en profondeur (*inlay*), une incrustation de surface (*onlay*), un placage ou des éléments de ceux-ci.

7. Procédé selon au moins l'une des revendications 1 à 6, dans lequel on revêt le corps de forme céramique solidifié au moyen de procédés connus en soi.
